# EUROPEAN PATENT APPLICATION

(11) **EP 3 660 386 A1**
(43) Date of publication of application: **03.06.2020**
(21) Application number: 18838095.0
(22) Date of filing: 26.07.2018
(51) Int. Cl.: F21S 2/00, F21V 14/02, G02B 26/08, F21V 15/01, F21S 9/02, F21V 23/04, F21V 19/00, F21V 1/22, F21Y 115/10

(54) **LIGHT SOURCE MODULE**

(30) Priority: 27.07.2017 KR 20170095693; 28.09.2017 KR 20170125706
(71) Applicant: Seoul Viosys Co., Ltd., Ansan-si, Gyeonggi-do 15429 (KR)
(72) Inventor: JEONG, Woong-Ki, Ansan-si Gyeonggi-do 15429 (KR); CHOI, Jae-Young, Ansan-si Gyeonggi-do 15429 (KR); HAN, Kyu-Won, Ansan-si Gyeonggi-do 15429 (KR); JANG, Seong-Tae, Ansan-si Gyeonggi-do 15429 (KR); JUNG, Sang-Wook, Ansan-si Gyeonggi-do 15429 (KR)
(74) Representative: Stolmár & Partner Patentanwälte PartG mbB
(86) International application number: PCT/KR2018/008486
(87) International publication number: WO 2019/022543

(57) **Abstract**

A light source module includes a light source unit including a substrate and a light-emitting device provided on the substrate; a base to fix the substrate and rotatable around a pivot axis; and a housing having a pivot ring surrounding the base, wherein the housing are coupled to the light source unit and the base.

## Description

### [TECHNICAL FIELD]

The present disclosure relates to a light source module, and more particularly to a rotatable light source module.

### [BACKGROUND ART]

Many devices or tools used in daily life employ a light source module as a lighting device or as a functional device such as a sterilization device. For example, an ultraviolet light source module for sterilizing tableware may be employed in kitchen furniture.

Accordingly, there is a need for a light source module that may effectively control an emitting angle of light.

### [DETAILED DESCRIPTION OF THE INVENTION]

### [TECHNICAL PROBLEM]

The present disclosure aims to provide a light source module that may effectively control a light emitting angle.

### [TECHNICAL SOLUTION]

A light source module in accordance with one embodiment of the present disclosure includes a light source unit including a substrate and a light-emitting device provided on the substrate; a base to fix the substrate and rotatable around a pivot axis; and a housing having a pivot ring surrounding the base, wherein the housing are coupled to the light source unit and the base.

In one embodiment of the present disclosure, the light-emitting device may have a orientation angle with respect to one direction, wherein the one direction may change within a predefined angle relative to the pivot axis.

In one embodiment of the present disclosure, the substrate may extend in one direction, wherein the base may be provided at least one of both ends of the substrate and may be coupled to the pivot ring.

In one embodiment of the present disclosure, the pivot ring may include a stopper to limit an angle of rotation of the base. The stopper may protrude from the pivot ring toward the base, wherein the base may have a rotatable movement-limiter, wherein a rotation range of the movement-limiter may be limited by the stopper. The movement-limiter may be partial-circle or arc shaped when viewed in a direction perpendicular to the pivot axis.

In one embodiment of the present disclosure, the pivot ring may have an opening, wherein the opening may be formed by removing a portion of the pivot ring, wherein the base may include a lever protruding in a direction perpendicular to the pivot axis and disposed within the opening. The lever may be movable within the opening.

In one embodiment of the present disclosure, the base may include a protrusion for rotation projecting outwards along the pivot axis.

In one embodiment of the present disclosure, the light source module may further include a protective tube containing the light source unit in the protective tube, wherein the protective tube protects the light source unit.

In one embodiment of the present disclosure, the light source module may further include a fastener mounted on the housing to fasten the light source module to an external component.

In one embodiment of the present disclosure, the fastener may include a hook engaging in a sliding manner. In one embodiment of the present disclosure, the fastener may include a clip.

In one embodiment of the present disclosure, the light source module may further include a printed circuit substrate positioned adjacent to the light source unit, wherein a controller to drive the light source unit may be mounted on the printed circuit substrate.

In one embodiment of the present disclosure, the light source module may further include a battery pack provided on one side of the housing to supply a power to the light source unit.

In one embodiment of the present disclosure, the battery pack may include a charger rechargeable by an external power source. The battery pack may include a USB port to supply a power to the charger.

In one embodiment of the present disclosure, the light source module may further include a motion sensor mounted on the housing. The light source module may further include an illuminance sensor mounted on the housing.

In one embodiment of the present disclosure, the substrate may have a plate shape having at least two planes, wherein the light-emitting device may be mounted on at least one of the planes. In one embodiment of the present disclosure, the substrate may be bent in different directions to define the at least two planes, wherein a plurality of light-emitting devices may be mounted on the at least two planes respectively. In one embodiment of the present disclosure, the substrate may be flexible.

In one embodiment of the present disclosure, the light source module may further include a controller configured to control a rotation of the light source unit and turn-on or turn-off of the light source unit. The controller may control the rotation of the light source unit and the turn on/off of the light source unit based on a result detected by at least one of the motion sensor or the illuminance sensor.

In one embodiment of the present disclosure, the light source module may be included in an illumination device. The illumination device may include at least one first light source member to emit visible light; at least one second light source member spaced apart from the first light source member and emitting light of a wavelength for sterilization; and a casing housing the first light source and second light source members, wherein the second light source member may include: a light source unit including a substrate and a light-emitting device provided on the substrate; a base to fix the substrate and rotatable around a pivot axis; and a housing having a pivot ring surrounding the base, wherein the housing are coupled to the light source unit and the base.

### [ADVANTAGEOUS EFFECTS OF THE INVENTION]

The present disclosure provides a light source module that may easily adjust a direction of light emitted from the light source.

### [DESCRIPTION OF THE DRAWINGS]

FIG. 1 is a perspective view showing a light source module according to one embodiment of the present disclosure.
FIG. 2A to FIG. 2E sequentially show a bottom view, a front view, a right side view, a left side view, a top view, and a rear view of a light source module according to one embodiment of the present disclosure.
FIG. 3 is an exploded perspective view of a light source module according to one embodiment of the present disclosure.
FIG. 4A and FIG. 4B are cross-sectional views showing one embodiment of a light-emitting device of FIG. 3.
FIG. 5A to FIG. 5C are cross-sectional views showing a light source according to one embodiment of the present disclosure.
FIG. 6A to FIG. 6C are cross-sectional views for explaining a rotation operation of a light source in a light source module according to one embodiment of the present disclosure.
FIG. 7A and FIG. 7B are a perspective view and an exploded perspective view showing a light source module according to one embodiment of the present disclosure.
FIG. 8A and FIG. 8B are a perspective view and an exploded perspective view showing a light source module according to one embodiment of the present disclosure.
FIG. 9A and FIG. 9B are a perspective view and an exploded perspective view of a light source module according to another embodiment of the present disclosure.
FIG. 10 is a perspective view of a state in which a bottom of the light source module shown in FIG. 9A and FIG. 9B is visible.
FIG. 11 is a block diagram showing a light source module according to one embodiment of the present disclosure.
FIG. 12 shows a kitchen illumination device according to one embodiment of the present disclosure.
FIG. 13 is a perspective view of a kitchen illumination device according to one embodiment of the present disclosure.

### [BEST MODE]

The present disclosure may be variously modified and may have various forms. Thus, specific embodiments are illustrated in the drawings and described in detail in a present specification. However, the specific embodiments are not intended to limit the present disclosure to a particular disclosure form. It should be understood that the present disclosure includes all changes, equivalents, and substitutes that fall within a spirit and scope of the present disclosure.

Hereinafter, exemplary embodiments of the present disclosure will be described in detail with reference to the accompanying drawings.

The light source module according to one embodiment of the present disclosure is directed to a light source module that may be used not only as an illumination device for illuminating a predefined region, but also as a sterilization device, depending on a light-emitting device as employed therein. The light source module according to one embodiment of the present disclosure may be employed in various external devices. The external device to which the light source module may be applied may refer to a device to which the light source module may be mounted. For example, the external device may include an indoor/outdoor electronic appliance such as a refrigerator, a washing machine, an air conditioner, an air purifier, and an insect trapping device, kitchen furniture including a sink or a slop-basin shelf, indoor furniture such as a wardrobe or a drawer box, or an outdoor device such as a drinking fountain.

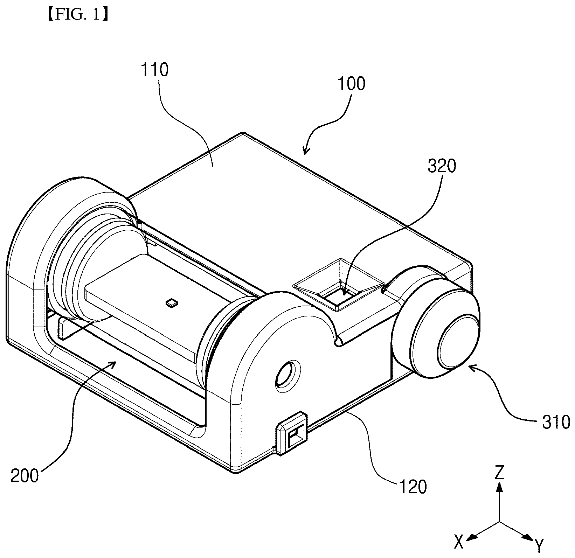

FIG. 1 is a perspective view showing a light source module according to one embodiment of the present disclosure.

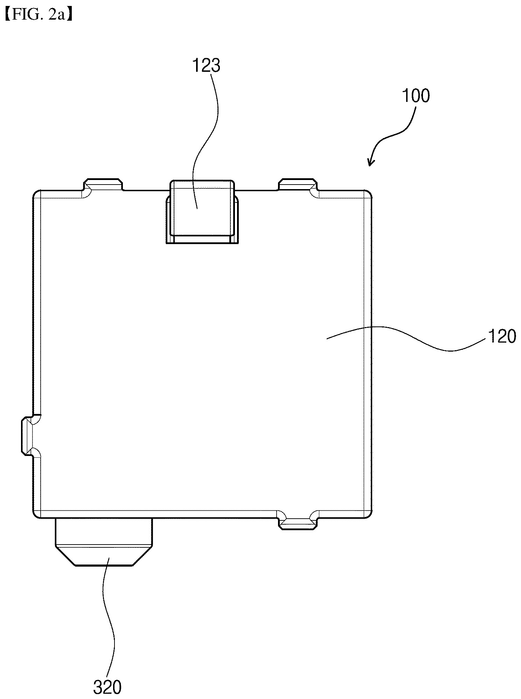

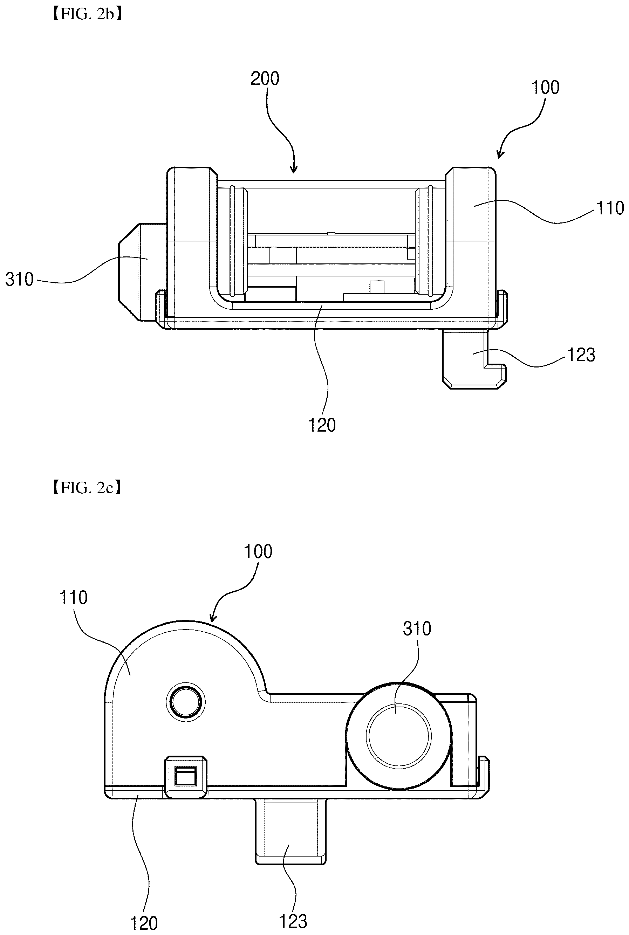

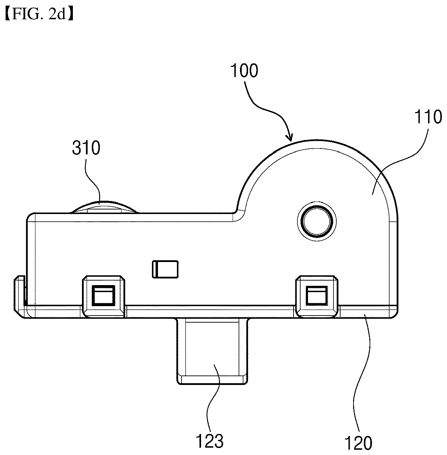

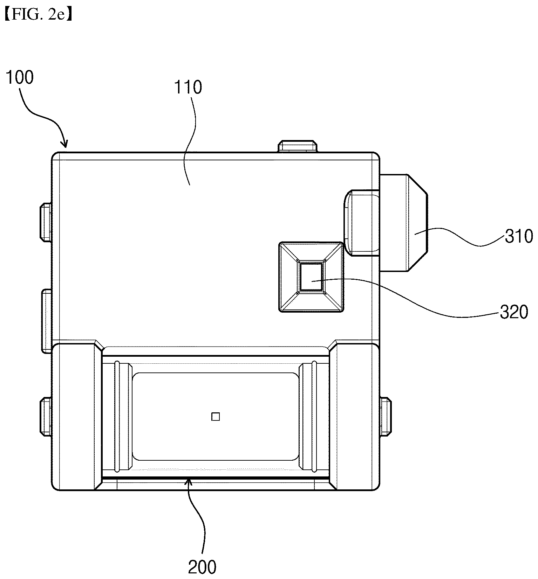

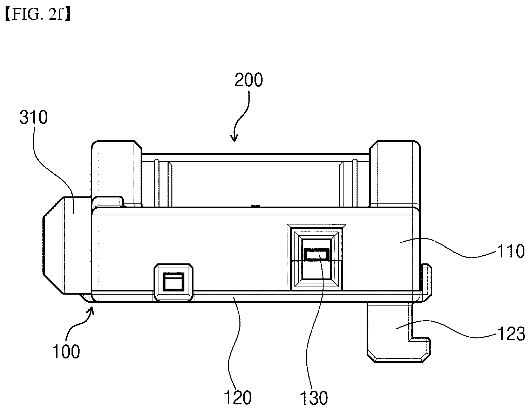

FIG. 2A to FIG. 2E sequentially show a bottom view, a front view, a right side view, a left side view, a top view, and a rear view of a light source module according to one embodiment of the present disclosure.

According to one embodiment of the present disclosure, an emission direction of light may vary depending on a mounting position or direction of a light source 200, and thus, in FIG. 1 and FIG. 2A to FIG. 2E, it is shown that the light from the light source 200 is emitted upwards (or in a z-axis direction). In following descriptions, a direction in which an x-axis is directed is referred to as a front direction, a direction in which a y-axis is directed is referred to as a lateral direction, and a direction in which the z-axis is directed referred to as an upward direction. However, the definition of the directions is merely for convenience of description. The direction may relatively vary depending on a direction in which the light source module is mounted on a specific device.

Referring to FIG. 1 and FIG. 2A to FIG. 2E, a light source module according to one embodiment of the present disclosure includes the light source 200 that emits light, and a housing 100 coupled with the light source 200.

The light source 200 includes a light-emitting device that emits light, and has a cylindrical shape as a whole. The light source 200 may be rotated around a center axis of a cylinder. Accordingly, light is generally emitted in the upward direction. However, the emission direction of the light may vary depending on whether the light source 200 is rotated.

The light source 200 is provided on one side of the housing 100. The housing 100 forms an appearance of the light source module, and provides a space within which the light source 200, a printed circuit board 400 for driving the light source 200, and wires may be placed.

The housing 100 has an approximately rectangular shape. However, the shape of the housing 100 is not limited thereto. Shapes of the light source 200 or other components accommodated in the housing 100 may vary depending on a size or shape of the light source 200 and the printed circuit board. In addition, although the housing 100 forms the appearance of the light source module, an additional casing or component may be further provided outside the light source module.

In one embodiment of the present disclosure, the housing 100 may be made of various materials. The housing 100 may be made of a polymer resin, but is not limited thereto. The housing 100 may be made of other durable materials as long as the housing 100 contains the light source 200. The housing 100 may be made of a metal material such as aluminum, stainless steel, or the like. Further, the housing 100 may be made of a single material or a combination of two or more materials. For example, a lower housing 120 may be made of a metal such as aluminum, while an upper housing 110 may be made of polymer resin or the like.

The housing 100 is provided with a pivot ring which is an annular structure supporting the light source 200 while surrounding both ends of the light source 200. The light source 200 is inserted into the pivot ring and fixed thereto in a rotatable form therein. Further, housing 100 is provided with a fastener for fastening the light source module to an external component.

The housing 100 may be provided as an assembly in which a plurality of parts are fastened to each other and are assembled with each other. In the present embodiment, the housing 100 includes the upper housing 110 and the lower housing 120 which are fastened to each other while facing each other. However, the housing 100 may include a larger number of parts.

The light source module may be provided with a fastener which fastens the light source module with an external device. The fasteners may be provided in various forms such as hooks, screws, adhesive tapes, Velcro, slide hooks, clips, and the like. In one embodiment of the present disclosure, the fastener is embodied as a hook 123.

The fastener may be fastened to various structures configured such that the fastener may be fastened thereto. For example, the fastener may be fastened in any direction, such as a vertical direction, a horizontal direction, other directions. The fastener may move in various directions depending on a shape of the structure. For example, the hook 123 may be mounted on a rail. In this case, the hook 123 may move in a horizontal or vertical direction of the light source module along the rail. In one embodiment of the present disclosure, a fixing member may be further provided around the fastener to fix the light source module so that the light source modules does not move after being fastened to the external structure. The fixing member may be provided on the light source module and may be provided on the external component.

In one embodiment of the present disclosure, the hook 123 as the fastener is formed on the lower housing 120. However, the present disclosure is not limited thereto. The hook 123 may be formed on any one of the upper housing 110 and lower housing 120 in consideration of a shape of the external component to be fastened thereto or in consideration of an arrangement therebetween.

The light source module according to one embodiment of the present disclosure may be equipped with various kinds of sensors. For example, the light source module may be equipped with a motion sensor 310 for detecting a user's movement and an illuminance sensor 320 for detecting an internal/ external illuminance. However, the sensors may optionally be omitted.

### [Modes]

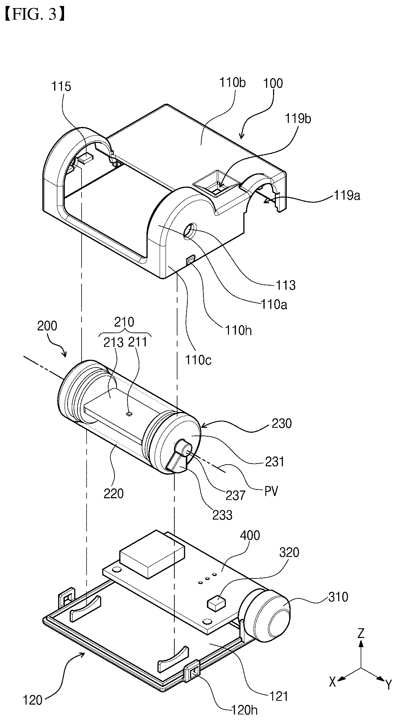

FIG. 3 is an exploded perspective view of a light source module according to one embodiment of the present disclosure. Hereinafter, the light source module will be described sequentially in a direction from a bottom to a top with reference to the exploded perspective view of FIG. 3.

The light source module may be assembled into a structure in which the light source 200 is disposed between the upper housing 110 and the lower housing 120. The printed circuit board 400 for controlling and driving the light source 200, the illuminance sensor 320 for detecting illuminance of external light, and the motion sensor 310 for detecting a user's movement are disposed between the upper housing 110 and the lower housing 120.

The lower housing 120 is generally provided in a quadrangular shape so that the light source 200, the printed circuit board 400, the illuminance sensor 320, and the motion sensor 310 may be disposed on a top surface of the lower housing 120.

The light source 200, the printed circuit board 400, the illuminance sensor 320, and the motion sensor 310 are mounted on the lower housing 120. The lower housing 120 may be provided with mounting protrusions for stably mounting thereon the light source 200, the printed circuit board 400, the motion sensor 310, and the like respectively. Further, the lower housing 120 may be provided with a fastener for fastening the lower housing 120 with the upper housing 110. In this embodiment, it is shown that a hook receiving portion 120h is formed as the fastener so that a hook 110h of the upper housing 110 may be inserted into and fastened with the hook receiving portion 120h.

The light source 200 includes a light source unit 210, a protective tube 220 to protect the light source unit 210, and a base 230 provided at each of both ends of the protective tube 220 and has a cylindrical shape as a whole. The light source 200 is rotatable around a pivot axis PV. The pivot axis PV is a longitudinal axis of a cylinder, and passes through a center of a cross section of the cylinder.

Light emitted by the light source 200 may have various wavelength bands. The light from light source 200 may have a visible wavelength band, an infrared wavelength band, or other wavelength bands. In one embodiment of the present disclosure, the light emitted from light source 200 may be selected from infrared light, visible light, ultraviolet light, etc., as desired. For example, for lighting purposes, the light source 200 may emit light in the visible light band. For the purpose of sterilization, the light source 200 may emit light in the ultraviolet wavelength band according to a target to be treated, for example, bacteria or germs.

In one embodiment of the present disclosure, the light source 200 may emit light in a wavelength band of about 100 nm to about 405 nm as a wavelength band in which light may sterilize microorganisms and the like. In one embodiment of the present disclosure, the light source 200 may emit light in a wavelength band of about 100 nm to about 280 nm. In another embodiment, the light of about 180nm to about 280nm wavelength band may be emitted from the light source 200. In still another embodiment, the light of about 250 nm to about 260 nm wavelength band may be emitted from the light source 200. Ultraviolet light of the wavelength band has a great bactericidal power. Thus, for example, when the ultraviolet light is irradiated at an intensity of 100 µW per 1 cm2, this may kill up to 99% of bacteria such as E. coli, diphtheria, dysentery. Further, the ultraviolet light in the wavelength band may kill food poisoning bacteria. Thus, the ultraviolet light may kill bacteria such as food poisoning pathogenic E. coli, Staphylococcus aureus, Salmonella weltedreden, Salmonella Typhumurium, Enterococcus faecalis, Bacillus cereus, Pseudomonas aeruginosa, Vibrio parahaemolyticus, Listeria monocytogenes, Yersinia enterocolitica, Clostridium perfringens, Clostridium botulinum, Campylobacter jejuni or Enterobacter sakazakii.

To emit the light as described above, the light source 200 may include at least one light-emitting device 211 that emits light. A type of the light-emitting device 211 is not particularly limited as long as the light-emitting device 211 emits desired light. For example, when the light source 200 emits light in the ultraviolet wavelength band, various light-emitting devices 211 that emit ultraviolet light may be used. A typical example of the light-emitting device 211 for emitting ultraviolet light may include a light-emitting diode. In another example, when the light source 200 emits light in other wavelength bands, other known light-emitting devices may be used.

When the light-emitting device 211 is used, the light-emitting device 211 may be mounted on a substrate 213. The substrate 213 and at least one light-emitting device 211 may constitute the light source unit 210.

The substrate 213 may be provided in a form extending in an elongate manner in a predefined direction, for example, in one direction. One light-emitting device 211 may be disposed on the substrate 213. However, the number of light-emitting devices 211 is not limited thereto. A plurality of light-emitting devices 211, for example, three light-emitting devices 211 may be arranged along a predefined direction, for example, said one direction. When the light source unit 210 includes the plurality of light-emitting devices 211, the light-emitting devices 211 may emit light of the same wavelength band or emit light beams of different wavelength bands respectively. For example, in one embodiment, all of the light-emitting devices 211 may emit light of the same or similar ultraviolet wavelength band. In another one embodiment, some light-emitting devices 211 may emit a portion of the ultraviolet wavelength band, while the remaining light-emitting devices 211 may emit another portion of the ultraviolet wavelength band.

When the light-emitting devices 211 have different wavelength bands, the light-emitting devices 211 may be arranged in various orders. For example, the light-emitting device 211 emitting light of a first wavelength band and the light-emitting device 211 emitting light of a second wavelength band different from the first wavelength band may be alternately arranged with each other.

The light source unit 210 is inserted into the protective tube 220 and is protected therein. The protective tube 220 is made of a transparent insulating material, and transmits the light emitted from the light-emitting devices 211 while protecting the light-emitting devices 211 and the substrate 213. The protective tube 220 may be made of a variety of materials, as long as the above function is realized. The material is not limited to a specific material. For example, the protective tube 220 may be made of quartz or a polymer organic material. In this connection, the polymer organic material may be selected in consideration of a wavelength of the light emitted from the light-emitting devices 211 because a wavelength in which the polymer organic material absorb or transmits light may vary depending on a type of a monomer thereof, a molding method, and a condition. For example, organic polymers such as poly(methylmethacrylate) (PMMA), polyvinylalcohol (PVA), polypropylene (PP), low density polyethylene (LDPE) may hardly absorb ultraviolet light, while an organic polymer such as polyester may absorb ultraviolet light.

The protective tube 220 may have a long cylindrical shape along an extending direction of the substrate 213, and both ends thereof are open. However, the shape of the protective tube 220 is not limited thereto. One end thereof may be opened while the other end thereof may be blocked. In this embodiment, a form in which both ends thereof are opened will be described as an example.

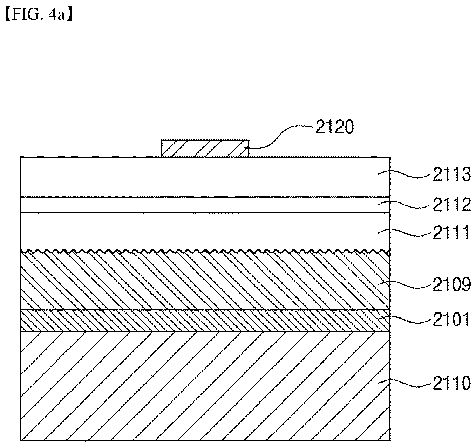

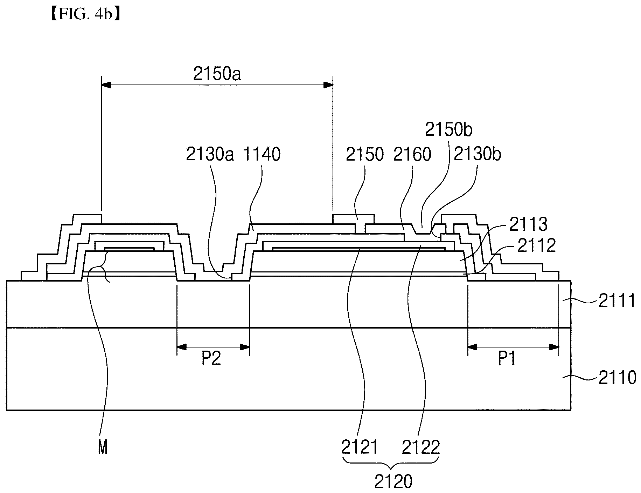

The light-emitting device 211 may be implemented in various forms. Thus, FIG. 4A and FIG. 4B are cross-sectional views showing one embodiment of the light-emitting device and show an example where the light-emitting device is implemented as a light-emitting diode.

The light-emitting diode may be configured in various forms, such as vertical type or flip type. FIG. 4A shows a vertical type light-emitting diode. FIG. 4B shows a flip type light-emitting diode. However, the configuration of the light-emitting diode is not limited thereto. Following drawings should be understood as one embodiment of the present disclosure.

Referring to FIG. 4A, the light-emitting diode includes a first conductive type semiconductor layer 2111, an active layer 2112, and a second conductive type semiconductor layer 2113. A substrate 2110 used as a first electrode, an adhesive layer 2101, and a reflective layer 2109 may be provided below the first conductive type semiconductor layer 2111 of the light-emitting diode, while a second electrode 2120 may be provided above the second conductive type semiconductor layer 2113.

The substrate 2110 may be made of a conductive material, and thus may be made of Si, GaAs, GaP, AlGaINP, Ge, SiSe, GaN, AlInGaN or InGaN or the like, or made of a single metal such as Al, Zn, Ag, W, Ti, Ni, Au, Mo, Pt, Pd, Cu, Cr or Fe, or an alloy thereof.

The second conductive type semiconductor layer 2113 may be disposed on the first conductive type semiconductor layer 2111. The active layer 2112 may be disposed between the first conductive type semiconductor layer 2111 and the second conductive type semiconductor layer 2113. Each of the first conductive type semiconductor layer 2111, the active layer 2112, and the second conductive type semiconductor layer 2113 may include a III-V based compound semiconductor, for example, a nitride-based semiconductor such as (Al, Ga, and In)N. The first conductive type semiconductor layer 2111 may contain first conductive type impurities such as Si, the second conductive type semiconductor layer 2113 may contain second conductive type impurities, for example, or vice versa.

In one embodiment of the present disclosure, the first conductive type semiconductor layer 2111 may be roughened. Accordingly, light generated from the active layer 2112 may be reflected from the roughened interface.

In one embodiment of the present disclosure, the reflective layer 2109 may be interposed between the first conductive type semiconductor layer 2111 and the light source substrate 2110. The reflective layer 2109 may be made of a highly reflective metal material such as silver (Ag) or aluminum (Al) or may be made of another metal having high reflectance or an alloy thereof with silver (Ag) or aluminum (Al).

In one example, the adhesive layer 2101 may be interposed between the reflective layer 2109 and the light source substrate 2110. The adhesive layer 2101 may improve adhesion between the light source substrate 2110 and the reflective layer 2109 to prevent the light source substrate 2110 from separating from the reflective layer 2109. In addition, although not shown, a diffusion barrier layer may be interposed between the adhesive layer 2101 and the reflective layer 2109. The diffusion barrier layer may maintain reflectivity of the reflective layer 2109 by preventing metal elements from diffusing from the adhesive layer 2101 or the light source substrate 2110 into the reflective layer 2109.

The second electrode 2120 is disposed on the second conductive type semiconductor layer 2113. Accordingly, light may be emitted by supplying current to the first conductive type semiconductor layer 2111 and the second conductive type semiconductor layer 2113 through the light source substrate 2110 used as the first electrode and the second electrode 2120

Referring to FIG. 4B, the light-emitting diode according to one embodiment of the present disclosure may include a mesa M including the first conductive type semiconductor layer 2111, the active layer 2112 and the second conductive type semiconductor layer 2113, and may further include first insulating layers 2130a and 2130b, a first electrode 1140, and a second insulating layer 2150. Furthermore, the substrate 2110 and the second electrode 2120 may be included in the light-emitting diode.

The substrate 2110 is not limited to a specific substrate as long as, on the substrate, the first conductive type semiconductor layer 2111, the active layer 2112, and the second conductive type semiconductor layer 2113 may be grown. For example, the substrate 2110 may include a sapphire substrate, a silicon carbide substrate, a gallium nitride substrate, an aluminum nitride substrate, a silicon substrate and the like. Each side surface of the substrate 2110 may be inclined. Accordingly, extraction of light generated from the active layer 2112 may be improved.

The second conductive type semiconductor layer 2113 may be disposed on the first conductive type semiconductor layer 2111. The active layer 2112 may be disposed between the first conductive type semiconductor layer 2111 and the second conductive type semiconductor layer 2113. Each of the first conductive type semiconductor layer 2111, the active layer 2112, and the second conductive type semiconductor layer 2113 may include a III-V based compound semiconductor, for example, a nitride-based semiconductor such as (Al, Ga, and In)N. The first conductive type semiconductor layer 2111 may contain first conductive type impurities such as Si, while the second conductive type semiconductor layer 2113 may contain second conductive type impurities, for example Mg, or vice versa. The active layer 2112 may include a multi-quantum well structure (MQM). When a forward bias is applied to the light-emitting diode, electrons and holes combine with each other in the active layer 2112 to emit light. The first conductive type semiconductor layer 2111, the active layer 2112, and the second conductive type semiconductor layer 2113 may be grown on the substrate 2110 using techniques such as metalorganic chemical vapor deposition (MOCVD) or molecular beam epitaxy (MBE).

The light-emitting diode may include at least one mesa M including the active layer 2112 and the second conductive type semiconductor layer 2113. The mesa M may include a plurality of protrusions. The plurality of protrusions may be spaced apart from each other. The present disclosure is not limited thereto. The light-emitting diode may include a plurality of mesas M spaced apart from each other. Each side surface of the mesa M may be formed in an inclined manner using techniques such as photoresist reflow. Each inclined side surface of the mesa M may improve light-emitting efficiency generated from the active layer 2112.

The first conductive type semiconductor layer 2111 has a first contact region P1 and a second contact region P2 exposed through the mesa M. Since the mesa M is formed by removing the active layer 2112 and the second conductive type semiconductor layer 2113 disposed on the first conductive type semiconductor layer 2111, a portion of the first conductive type semiconductor layer 2111 except the mesa M becomes a contact region as an exposed top surface of the first conductive type semiconductor layer 2111.

The first electrode 1140 may contact the first contact region P1 and the second contact region P2 and thus may be electrically connected to the first conductive type semiconductor layer 2111. The first contact region P1 may be disposed around the mesa M and along a perimeter of the first conductive type semiconductor layer 2111. Specifically, the first contact region P1 may be disposed along a perimeter of a top surface of the first conductive type semiconductor layer and between the mesa M and a side surface of the light-emitting diode. The second contact region P2 may be at least partially surrounded by the mesa M.

The second electrode 2120 may be disposed on the second conductive type semiconductor layer 2113, and may be electrically connected to the second conductive type semiconductor layer 2113. The second electrode 2120 may include a reflective metal layer 2121 and may further include a barrier metal layer 2122. The barrier metal layer 2122 may cover a top surface and side surfaces of the reflective metal layer 2121. For example, the barrier metal layer 2122 may be formed to cover the top surface and side surfaces of the reflective metal layer 2121 by forming a pattern of the reflective metal layer 2121 and forming the barrier metal layer 2122 thereon. For example, the reflective metal layer 2121 may be formed by depositing and patterning an Ag, Ag alloy, Ni/Ag, NiZn/Ag, TiO/Ag layer.

In one example, the barrier metal layer 2122 may be formed of a Ni, Cr, Ti, Pt, Au layer, or a stack thereof. Specifically, the barrier metal layer 2122 may be embodied as a stack of Ni/Ag/[Ni/Ti]2/Au/Ti layers sequentially on a top surface of the second conductive type semiconductor layer 2113. More specifically, at least a portion of the barrier metal layer 2122 on the top surface of the second electrode 2120 may include a 300 Å thick Ti layer. When a portion of the barrier metal layer 2122 in contact with the first insulating layer on the top surface of the second electrode 2120 is formed of a Ti layer, adhesion between the first insulating layers 2130a and 2130b and the second electrode 2120 may be improved, so that reliability of the light-emitting diode may be improved.

An electrode protective layer 2160 may be disposed on the second electrode 2120. The electrode protective layer 2160 may be made of the same material as the first electrode 1140, but is not limited thereto.

The first electrode 1140 may be formed on the mesa M, and may have the same shape as a shape of the mesa M. The first insulating layers 2130a and 2130b may be disposed between the first electrode 1140 and the mesa M. Accordingly, the first electrode 1140 and mesa M may be insulated from each other by the first insulating layers 2130a and 2130b. Further, the first electrode 1140 and the second electrode 2120 may be insulated from each other by the first insulating layers 2130a and 2130b. The first insulating layers 2130a and 2130b may partially expose the first contact region P1 and the second contact region P2. In detail, the first insulating layers 2130a and 2130b may expose a portion of the second contact region P2 through an opening. The first insulating layers 2130a and 2130b may cover only a portion of the first contact region P1 and between a perimeter of the first conductive type semiconductor layer 2111 and the mesa M, so that at least a portion of the first contact region P1 may be exposed.

The first insulating layers 2130a and 2130b may be disposed on the second contact region P2 and along a perimeter of the second contact region P2. At the same time, the first insulating layers 2130a and 2130b may be disposed closer to the mesa M than to a region where the first contact region P1 and the first electrode 1140 contact each other.

The first insulating layers 2130a and 2130b may have an opening that exposes the second electrode 2120. The second electrode 2120 may be electrically connected to a pad or a bump through the opening.

Although not shown, in a plan view, a region where the first contact region P1 and the first electrode 1140 contact each other is disposed along an entire perimeter of a top surface of the first conductive type semiconductor layer 2111. Specifically, the region where the first contact region P1 and the first electrode 1140 contact each other may be disposed to be adjacent to all of four side surfaces of the first conductive type semiconductor layer 2111 and may surround the mesa M completely. In this case, since a region where the first electrode 1140 and the first conductive type semiconductor layer 2111 contact each other may increase, current flowing from the first electrode 1140 to the first conductive type semiconductor layer 2111 may be more effectively distributed, so that a forward voltage may be further reduced.

In one embodiment of the present disclosure, the first electrode 1140 and the second electrode 2120 of the light-emitting diode may be mounted on the substrate 213 directly or indirectly via a pad.

For example, when the light-emitting diode is mounted on the substrate 213 via the pad, two pads may be disposed between the light-emitting diode and the substrate 213. The two pads may be in contact with the first electrode 1140 and the second electrode 2120, respectively. For example, the pad may be a solder or an eutectic metal, but may not be limited thereto. For example, AuSn may be used as the eutectic metal.

In another example, when the light-emitting diode is mounted directly on the substrate 213, the first electrode 1140 and the second electrode 2120 of the light-emitting diode may be bonded directly to a wire on the substrate 213. In this case, a bonding material may include an adhesive material having conductive properties. For example, the bonding material may include at least one of conductive materials such as silver (Ag), tin (Sn), and copper (Cu). However, this is exemplary. The bonding material may include various materials having conductivity.

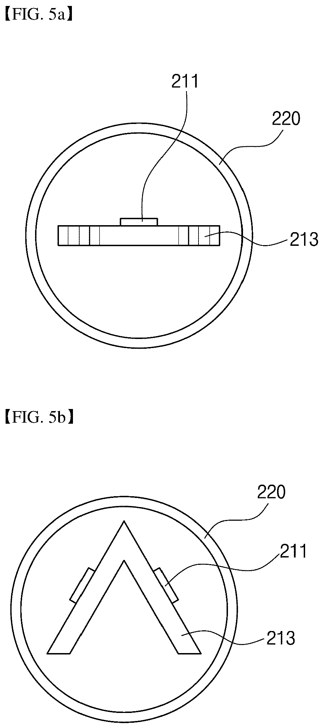

In one embodiment of the present disclosure, the light source unit 210 contained in the protective tube 220 may be provided in various forms. FIG. 5A to FIG. 5C are cross-sectional views of the light source 200 according to embodiments of the present disclosure.

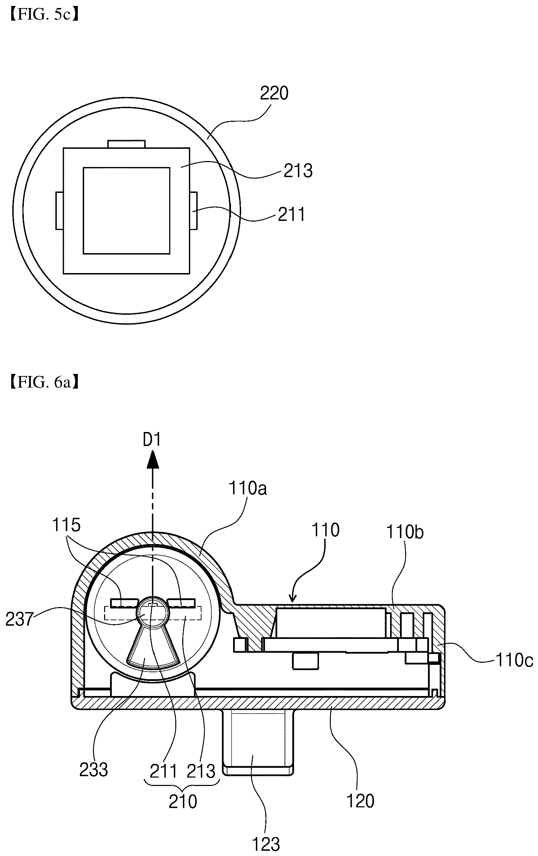

Referring to FIG. 5A to FIG. 5C, the substrate 213 of the light source unit 210 may have one flat plate, as shown in FIG. 5A, but may have multiple flat plates, as shown in FIG. 5B or FIG. 5C. That is, the substrate 213 of the light source unit 210 may be provided as a plurality of flat plates. The light-emitting device 211 may or may not be mounted on the plates. For example, as shown in FIG. 5B, the substrate 213 may have two flat plates that are formed by bending a single flat plate. The light-emitting device 211 may be mounted on each of two outwards-facing planes of the substrate 213. Alternatively, as shown in FIG. 5C, the substrate 213 may be formed of four flat plates such that the substrate 213 has a quadrangular cross section. The light-emitting device 211 may be mounted on each of three of four outwards-facing planes of the substrate 213. Further, the substrate 213 may be made of a flexible material. In this case, the substrate 213 may be bent in various forms.

As such, when the substrate 213 is bent into a curved shape or is simply bent, the light-emitting devices 211 mounted on a top surface of the substrate may emit light beams in different directions. Accordingly, when the light emitted from the light-emitting device 211 of the light source 200 emits in a predefined direction at a predefined directivity angle, an emission range of the light may be limited. When the light-emitting devices 211 emit light beams in different directions, the emission range of the light may be extended even though the light source 200 is rotated.

Referring back to FIG. 3, the base 230 is provided at each of both ends of the protective tube 220. The base 230 includes a base body 231 having a diameter at which the base body is capable of encapsulating the opened portion of the protective tube 220 at each of both ends of the protective tube 220, an inserted protrusion 237 and a movement-limiter 233 protruding from the base body 231.

The base body 231 may have a step portion having a diameter corresponding to an inner circumferential surface of the protective tube 220. The step portion is inserted into the protective tube 220 to separate an inner space of the protective tube 220 from an external space.

In an inner surface of the base body 231 facing an inner space of the protective tube 220, a groove is defined into which the substrate 213 of the light source unit 210 is inserted. One end of the substrate 213 is inserted into the groove so that the light source unit 210 is stably fixed in the protective tube 220. On an outer surface of the base body 231, the inserted protrusion 237 for engagement with the upper housing 110 and the movement-limiter 233 for limiting an angle of rotation of the light source 200 are formed. The inserted protrusion 237 is formed on the pivot axis PV of the light source 200. That is, a projection position of the inserted protrusion 237 coincides with the pivot axis PV. That is, the projection direction of the protrusion 237, and an extension direction of the pivot axis PV may be same. The inserted protrusion 237 and the movement-limiter 233 may protrude outwardly from the outer surface of the base body 231 and may be formed integrally with the base body 231 and thus may not be separated therefrom.

Although not shown, the base body 231 may be further provided with an outlet for wires or an electrode for supplying power to the substrate 213 and the light-emitting device 211 inside the protective tube 220.

On the lower housing 120, the printed circuit board 400 is disposed adjacent to the light source 200. In the present embodiment, the light source 200 and the printed circuit board 400 are disposed to be spaced apart from each other in a plan view, but are not limited thereto. The light source 200 and the printed circuit board 400 may overlap each other. That is, the printed circuit board 400 may be provided between the lower housing 120 and the light source 200.

The printed circuit board 400 is provided on one side of the light source 200 and is connected to the light source 200 via a wire. A controller for driving the light source 200 is formed on the printed circuit board 400. The power is applied to the light source 200 through the printed circuit board 400. Turn on/off of the light source 200 is controlled according to a signal from the controller. In the present embodiment, the printed circuit board 400 is illustrated as having a rectangular shape, but is not limited thereto. The printed circuit board 400 may be manufactured in various shapes and a size thereof may vary. According to the embodiment, in this case, when a space occupied by the printed circuit board 400 in the housing 100 becomes smaller, a shape and size of the housing 100 may vary.

Further, the illuminance sensor 320 and the motion sensor 310 are provided on the lower housing 120. The illuminance sensor 320 and the motion sensor 310 may be electrically connected to the controller of the printed circuit board 400 via a separate wire. In the present embodiment, the illuminance sensor 320 may be positioned to face in the z-axis direction, while the motion sensor 310 may be positioned to face in the y-axis direction. Accordingly, the illuminance sensor 320 may be provided on the printed circuit board 400, while the motion sensor 310 may be provided on a y-axis side surface of the printed circuit board 400. However, the positions where the illuminance sensor 320 and the motion sensor 310 are disposed in the lower housing 120 may be variously changed.

The Illuminance sensor 320 may be provided in a singular or plural number, and may detect illuminance based on light from another external light source and/or illuminance based on light from the light source of the light source module. That is, the Illuminance sensor 320 may detect illuminance resulting from external light sources such as external lighting and sunlight, or illuminance resulting from the light-emitting diode of the light source module.

The motion sensor 310 detects movement of the user. However, the motion sensor 310 may detect movement of a moving object, for example, a pet, in addition to the user. The motion sensor 310 may be implemented in various ways. For example, the motion sensor 310 may be a thermal based motion sensor.

In one embodiment of the present disclosure, the illuminance sensor 320 and the motion sensor 310 have been described by way of example. However, for convenience of description, other types of sensors may be further included depending on circumstances.

The upper housing 110 covers a portion of the light source 200 and includes each pivot ring 110a to fix the light source 200, a cover 110b to cover the printed circuit board 400, and each lateral portion 110c to cover each side surface thereof. The upper housing 110 is absent in a region where light is emitted from the light source 200.

Each pivot ring 110a has a ring shape to stably support the light source 200 while allowing rotation of the light source 200. The light source 200 is rotatably secured at both ends thereof to both pivot rings 110a respectively. Each pivot ring 110a is absent in a region in which light is emitted and covers an outer circumference of each base 230 of the light source 200, and has an inner diameter corresponding to a diameter of the outer circumference surface of each base 230. That is, the inner diameter of each pivot ring 110a may have a diameter equal to or slightly larger than an outer diameter of each base 230 of the light source 200 such that each base 230 of the light source 200 may rotate therein.

The upper housing 110 and the lower housing 120 provides a space in which the light source 200 is seated. In particular, each base 230 of the light source 200 is disposed in each pivot ring 110a of the upper housing 110. The outer circumferential surface of an upper part of each base 230 is covered by each pivot ring 110a of the upper housing 110, while a lower part of the base 230 is supported by a protrusion of the lower housing 120. The base 230 of the light source 200 has a circular cross section to facilitate rotation thereof. The pivot ring 110a which covers the base 230 has a circular cross section.

The cover 110b is provided on the light source 200, the printed circuit board 400 and the like, and covers a region where the pivot ring 110a is not formed.

The cover 110b has an illuminance sensor opening 119b for exposing a part of the illuminance sensor 320 to an outside so that the illuminance sensor 320 may detect an external light source.

The lateral portion 110c covers a region between the pivot ring 110a and the lower housing 120 and a region between the cover 110b and the lower housing 120. The lateral portion 110c provided between the cover 110b and the lower housing 120 may be provided with a motion sensor opening 119a so that the motion sensor 310 may be mounted therein. The lateral portion 110c provided between the pivot ring 110a and the lower housing 120 may be provided with a fastener for engaging with the light source 200. In this embodiment, the lateral portion 110c has a receiving hole 113 at a position corresponding to the pivot axis PV of the light source 200. In the receiving hole 113, the inserted protrusion 237 of the base 230 of the light source 200 is inserted, so that the lateral portion 110c of the lower housing 120 and the light source 200 are fastened to each other. An inner diameter of the receiving hole 113 is similar to or slightly larger than an outer diameter of the inserted protrusion 237 so that the inserted protrusion 237 may be inserted and rotated.

The lateral portion 110c has a stopper 115 that limits a rotational position of the movement-limiter 233 of the base 230 and is disposed adjacent to the receiving hole 113. As long as the stopper 115 may limit the rotational position of the movement-limiter 233, the stopper may have various shapes. In this embodiment, the stoppers 115 are embodied as two bars symmetrically provided on both sides of the receiving hole respectively.

In one embodiment of the present disclosure, the pivot ring 110a, the cover 110b, and the lateral portion 110c of the upper housing 110 may be integral with each other and are not separated from each other but are not limited thereto. A portion of the upper housing 110 may be provided separately and assembled together with a remaining portion thereof. Further, some of the pivot ring 110a, the cover 110b, and the lateral portion 110c may be provided on the lower housing 120 instead of the upper housing 110 and may be provided as one piece integral with or assembled with the lower housing 120.

In the present embodiment, wires for electrical connections between the light source 200, the printed circuit board 400, the illuminance sensor 320, and the motion sensor 310, and terminals or switches connected to the wires, and the like may be further provided. Further, the upper housing 110 and lower housing 120 may be further equipped with openings for withdrawing the wires, and additional components such as fastening structures for coupling with other components. For example, the upper housing 110 may be provided with a wire outlet 130 for withdrawing internal wires to the outside. Alternatively, the upper housing 110 or the lower housing 120 may be provided with a terminal for applying an external signal or supplying a power. A position or shape of the terminal may be variously modified.

FIG. 6A to FIG. 6C are cross-sectional views for explaining rotation of the light source 200 in the light source module according to one embodiment of the present disclosure. In the drawings, for convenience of description, some components are omitted and the housing 100 and the base 230 are mainly shown. Further, the substrate 213 and the light-emitting device 211 of light source unit 210 are indicated by dotted lines. In the drawings, the actual light emitted from the light source unit 210 may be emitted in a direction perpendicular to a plane of the substrate 213 and, further, at a predefined angle relative to the direction perpendicular thereto. However, for convenience of description, an emission direction of the light emitted from the light source unit 210 is indicated to be perpendicular to the substrate 213.

Referring to FIG. 6A to FIG. 6C, the light source 200 according to one embodiment of the present disclosure may be rotated along the pivot axis PV, which is the central axis in the longitudinal direction. When the light source 200 is rotated, the light source unit 210 mounted therein may rotate to the same degree as a rotation degree of the base 230.

As described above, the base 230 may be provided with the movement-limiter 233 protruding toward the lateral portion 110c of the upper housing 110 along the direction of the light source 200. The movement-limiter 233 may rotate around the inserted protrusion 237 located on the pivot axis PV of the light source 200 as the light source 200 rotates.

In one embodiment of the present disclosure, a pair of stoppers 115 limiting the angle of rotation of the light source 200 may be provided such that the direction of the light emitted from the light source unit 210 may change within an angle range relative to the pivot axis PV. In one embodiment of the present disclosure, the light-emitting device 211 of the light source 200 does not have to emit light toward the lower housing 120. Thus, the stoppers may limit the rotation angle of the light source 200 so that light is emitted only in the upward direction. The stopper 115 is placed at a position which the stopper blocks the rotation of the movement-limiter 233 to limit the range of the rotation of movement-limiter 233. In this connection, the movement-limiter 233 is provided between the pair of stopper 115. Thus, when the movement-limiter 233 rotates clockwise and counterclockwise, the rotation angle thereof is limited in such a way that the movement-limiter 233 is blocked by each of the stoppers 115. The position of the stopper 115 may vary according to the emission direction of the emitted light from the light source 200 and the directivity angle of the emitted light.

In one embodiment of the present disclosure, the shapes of the movement-limiter 233 and the stopper 115 may be variously modified. For example, the movement-limiter 233 may extend radially from the inserted protrusion 237 as shown in a cross section view. A surface of the movement-limiter 233 contacting the pivot ring 110a may have a shape of a partial circle, for example, a semicircle or an arc, to facilitate rotation thereof. However, the shape of the movement-limiter 233 is not limited thereto. The shape of the movement-limiter 233 may have any shape as long as the movement thereof may be blocked by the stopper 115. The shape of the cross section thereof may have various shapes such as a square, circle, ellipse, and the like. Further, the stopper 115 may be embodied as a pair of bars as shown, but may not be limited to this configuration as long as the stopper may prevent the rotation of the movement-limiter 233.

FIG. 6A shows that the light source 200 is not rotated. In this connection, the substrate 213 of the light source unit 210 is parallel to a bottom portion 121 of the lower housing 120 in a cross section view. The light source unit 210 emits light vertically upwards from the top surface of the light source module, that is, in a first direction D1.

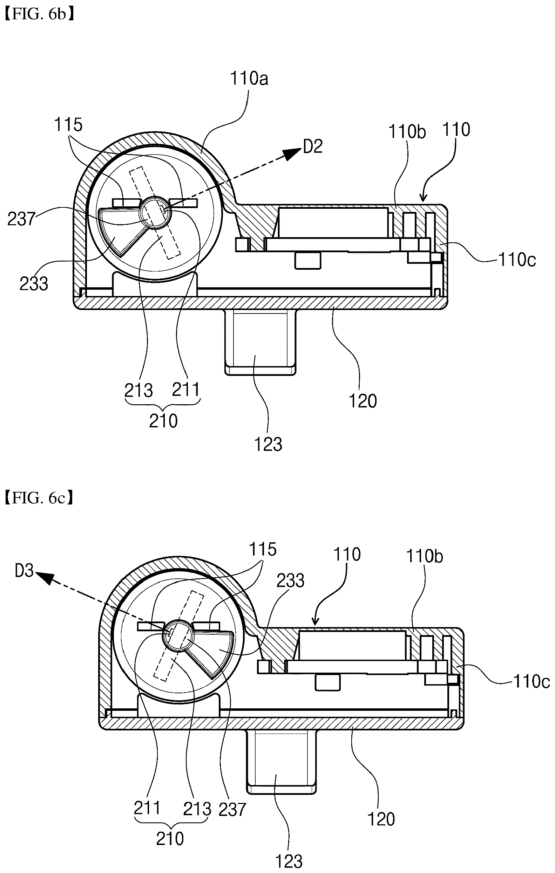

FIG. 6B shows that the light source 200 is rotated clockwise to a maximum extent. In this connection, the substrate 213 of the light source unit 210 is inclined relative to the bottom portion 121 of the housing 100 in a cross section view. The light source 200 may rotate clockwise until the movement-limiter 233 is stopped by the stopper 115. At this time, the light source unit 210 emits light in a direction tilted clockwise relative to an upward direction perpendicular to a top surface of the light source module, that is, in a second direction D2.

FIG. 6C shows that the light source 200 is rotated counterclockwise to a maximum extent. In this connection, the substrate 213 of the light source unit 210 is inclined in an opposite direction to the inclined direction in FIG. 6B relative to the bottom of the housing 100. The light source 200 may rotate counterclockwise until the movement-limiter 233 is stopped by the stopper 115. At this time, the light source unit 210 emits light in a direction tilted counterclockwise relative to an upward direction perpendicular to the top surface of the light source module, that is, in a third direction D3.

Eventually, according to one embodiment of the present disclosure, the emission direction of light from the light source 200 may be within an angle range between the third direction D3 and the second direction D2. In this connection, it should be appreciated that because the second direction D2 and the third direction D3 are based only on the direction perpendicular to the plane of the substrate 213 of the light source unit 210, an angle at which light is actually emitted is greater than the angle between the second direction D2 and the third direction D3.

In the light source module according to one embodiment of the present disclosure having the above structure, the light source 200 may be rotatable. Accordingly, the direction of the light emitted from the light source 200 may be adjusted in various ways. In particular, the user may easily adjust the light emission angle and range by rotating the light source 200 directly. Accordingly, light may be selectively irradiated to a target region, thereby to obtain an efficient light treatment result according to the light irradiation.

Further, the light source module according to one embodiment of the present disclosure may emit light in a wide range while having a compact size. Thus, the light source module may be easily carried and may selectively supply light of a predefined wavelength band in a wide or narrow range efficiently. In particular, the light source module according to one embodiment of the present disclosure includes the fastener such as a hook or a clip, and thus may be easily coupled to other devices. As a result, the light source module may be used regardless of various devices or locations.

In one embodiment of the present disclosure, the light source module may further employ a rotating member to allow the user to easily rotate the light source 200.

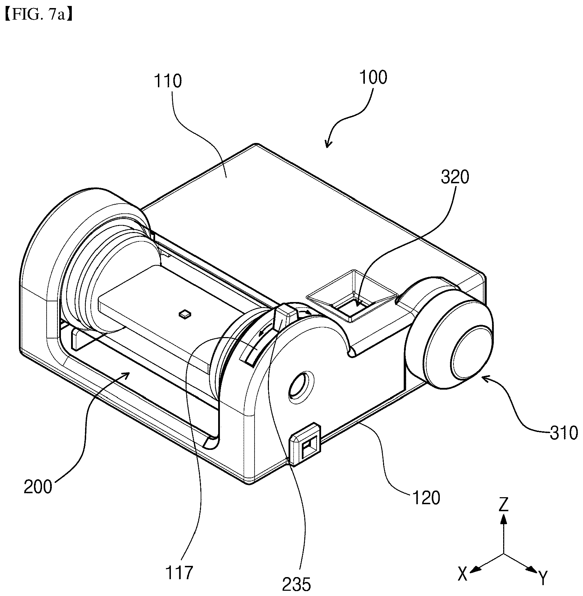

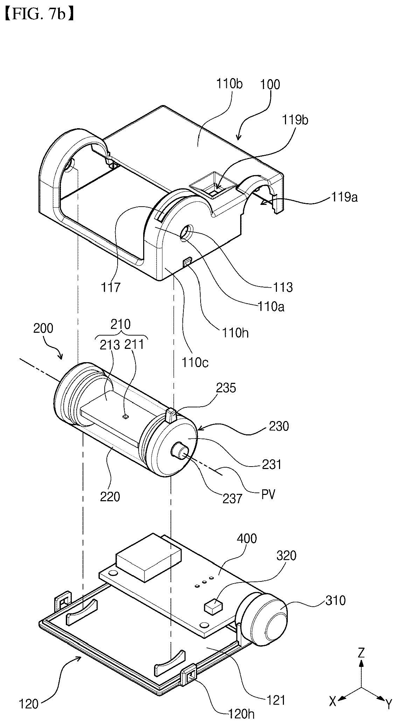

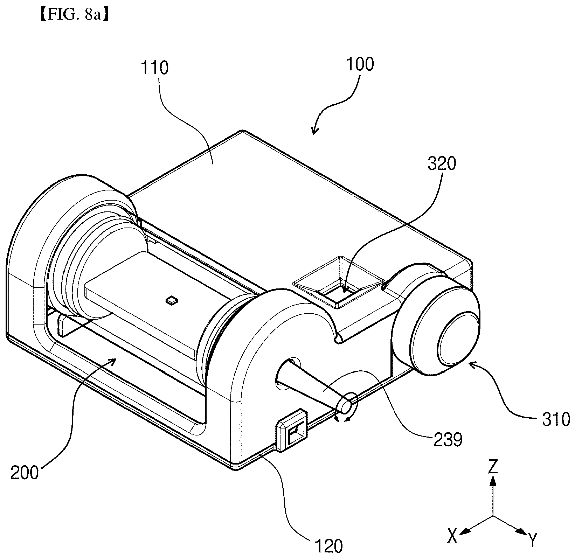

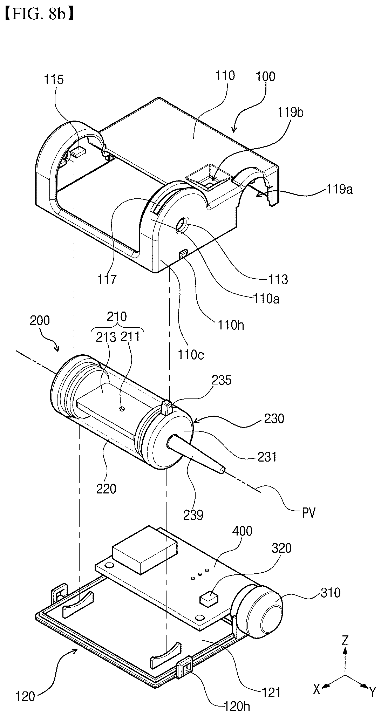

FIG. 7A and FIG. 7B, and FIG. 8A and FIG. 8B show a light source module according to one embodiment of the present disclosure. FIG. 7A and FIG. 8A are perspective views of a light source module employing a rotating member. FIG. 7B and FIG. 8B are exploded perspective views of FIG. 7A and FIG. 8A, respectively. In following embodiments, following descriptions will focus on differences from the above descriptions in order to avoid duplication of descriptions. The components as not described are the same as in the above-described embodiment.

Referring to FIG. 7A and FIG. 7B, the base 230 of the light source 200 is provided with a rotating lever 235. The rotating lever 235 protrudes from an outer circumferential surface of the base 230. The pivot ring 110a of the upper housing 110 has an opening 117 defined therein by removing a portion of the pivot ring 110a. The rotating lever 235 passes through the opening 117. The opening 117 may be elongate along the pivot ring 110a in a corresponding manner to the outer circumference of the base 230. The rotating lever 235 is movable in the opening 117 along a direction of an extension of the opening 117.

The user may easily rotate the light source 200 by moving the rotating lever 235 in the opening 117. In this connection, the movement range of the rotating lever 235 is limited by a length in an extending direction of the opening 117. Thus, it is not necessary to provide a separate stopper 115 on the lateral portion 110c of the upper housing 110.

Referring to FIG. 8A and FIG. 8B, the base 230 of the light source 200 may be provided with a protrusion for rotation 239 extending outwardly at a position corresponding to the inserted protrusion 237. The protrusion for rotation 239 protrudes from a side surface of the base 230 facing the lateral portion 110c of the upper housing 110, and extends through the receiving hole 113 of the upper housing 110 to the outside of the lateral portion 110c.

The user may easily rotate the light source 200 by rotating the protrusion for rotation 239 protruding outwardly. In this connection, there is no means for controlling a rotation angle of the protrusion for rotation 239. Thus, a separate stopper 115 may be required on the lateral portion 110c of the upper housing 110. However, the stopper 115 to limit the angle may be installed at a position between the protrusion for rotation 239 and the lateral portion 110c of the upper housing 110. In this case, the separate stopper 115 need not be provided on the lateral portion 110c of the upper housing 110.

As described above, the light source module according to one embodiment of the present disclosure allows the user to easily rotate the light source 200 to effectively provide the light to a target site. In this connection, in the above-described embodiments, the rotation of the light source 200 is caused by direct manipulation of the user. However, the concept of the present disclosure is not limited thereto. Although not shown separately, an automatic rotating member, for example, a rotary motor may be mounted on the pivot ring or base. In this case, the automatic rotating member may easily rotate the light source when the user simply presses a button. Further, when the automatic rotating member is connected to the controller, the rotation of the module may be automatically controlled according to a signal from the controller.

The light source module according to one embodiment of the present disclosure may further include an additional component such as a battery pack 500 for supplying a power.

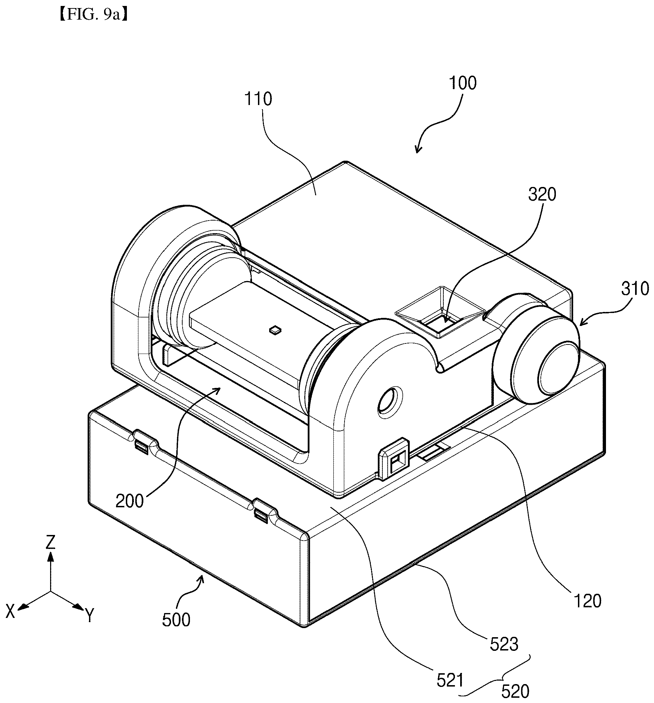

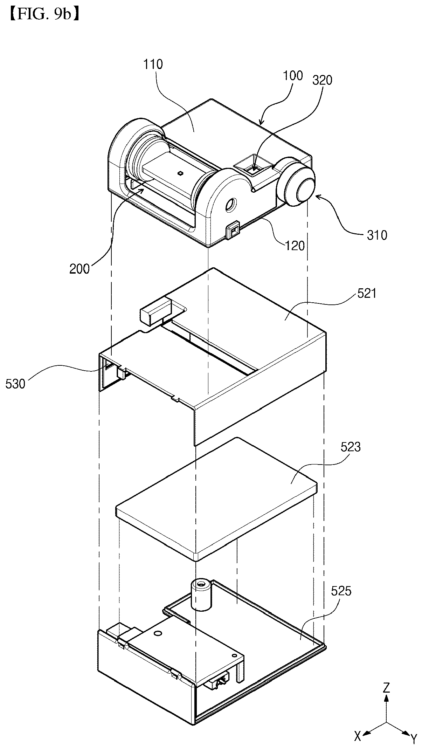

FIG. 9A and FIG. 9B are a perspective view and an exploded perspective view of a light source module according to another embodiment of the present disclosure.

Referring to FIG. 9A and FIG. 9B, on a rear face of the lower housing 120 below the upper housing, the battery pack 500 may be mounted to provide a power to the light source 200 and a driver of the printed circuit board 400.

The battery pack 500 includes a battery 510 and a battery cover 520 surrounding the battery 510. The battery cover 520 includes an upper cover 521 provided at a top of the battery 510 and connected to the lower housing 120 and a lower cover 523 provided at a bottom of the battery 510. Either the upper cover 521 or the lower cover 523 may cover a side surface of the battery 510.

The battery 510 is configured to provide a power to the light source 200, the driver, the sensor, and the like and is not limited to a specific type. The battery 510 includes a battery which may be charged by an external power source as well as a general battery such as a dry cell or a mercury battery. When the battery 510 is a rechargeable charger, the opening 117 for a charging port for charging the battery 510 may be formed in the upper cover 521 or the lower cover 523. The charging port may be provided in various forms or may be a USB port 530.

Although not shown separately, separate wires may connect the battery 510 with the light source 200, the driver, the motion sensor 310, the illuminance sensor 320, and the like.

In the light source module equipped with the battery pack 500, it is not necessary to apply a power through a power wire to the light source 200 and the driver. Thus, the light source module is suitable for use in a portable application or in a separate region from a power source. A rear face or a side of the battery pack 500 may be provided with a fastener for fastening the light source module to other furniture or devices when carrying the light source module.

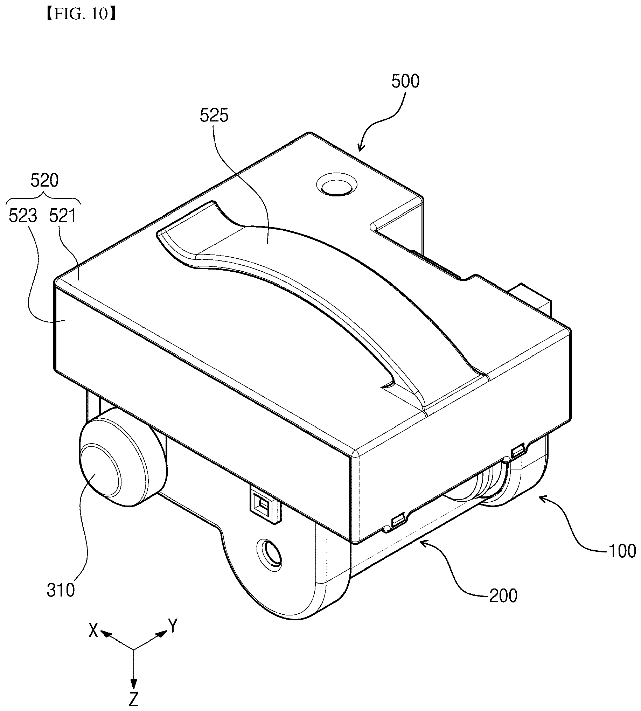

FIG. 10 is a perspective view of a state in which a bottom of the light source module shown in FIG. 9A and FIG. 9B is visible.

As shown in FIG. 10, in one embodiment of the present disclosure, a fastener may be provided at a bottom of the light source module. The fastener may be a clip 525. The clip 525 may be integrally formed with a portion of the battery cover 520 and may not be separated therefrom.

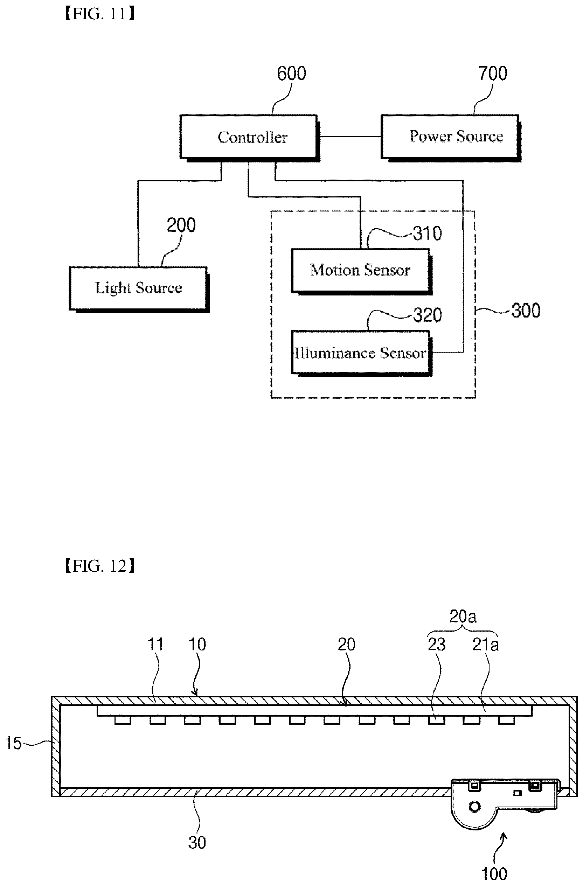

However, the fastener may not be limited thereto and may be provided in various forms such as bars, hooks, screws, adhesive tapes, Velcro, and slide hooks. The light source module according to one embodiment of the present disclosure may be driven in various ways. FIG. 11 is a block diagram illustrating a light source module according to one embodiment of the present disclosure.

Referring to FIG. 11, the light source module according to one embodiment of the present disclosure may include the light source 200 for emitting visible light, a sensor module 300 for detecting the user's motion or the illuminance of external light, and a controller 600. The controller 600 is connected to each of the light source 200 and the sensor module 300 to control each of the light source 200 and the sensor module 300. A power source 700 is connected to the controller 600. The power source 700 supplies a power to the light source 200 and the sensor module 300. Alternatively, power may be applied from a separate external wire or from a battery pack to the light source 200 and the sensor module 300.

The controller 600 may include a illuminance senor for detecting a user's motion and/or for detecting illuminance of external light.

The controller 600 may control the turn on/off of the light source 200 according to a sensed result of at least one of the illuminance sensor 320 and the motion sensor 310. In addition, the controller 600 may control whether the light source 200 is rotated and a rotation angle thereof when a separate automatic rotating member that may rotate the light source 200 is employed.

The motion sensor 310 may detect whether the user is moving. The light source 200 may be turned on/off based on the motion data of the user sensed by the motion sensor 310. For example, when the light source module emits ultraviolet light and when the user movement is detected, the controller 600 may turn off the light source 200. When there is no user movement, the light source 200 may be driven. Alternatively, when the motion sensor 310a detects that a distance between the light source module and the user reaches a preset limit approach distance, the controller may turn off the light source 200 or rotate the light source 200 to an angle at which light does not reach the user.

The illuminance sensor 320 may detect illuminance of ambient external light. The controller 600 may turn the light source 200 on or off based on data from the illuminance sensor 320. For example, the controller may turn off the light source 200 during daytime with high illuminance or may turn on the light source 200 at night with low illuminance. In particular, when the light source module emits ultraviolet light, the controller may not drive the light source module. This is because the ultraviolet light may be otherwise applied to the user when the light source module is turned on regardless of the day when the user is active or the nighttime.

In one embodiment of the present disclosure, when the light source unit of the light source module has a structure as shown in FIG. 5B or FIG. 5C described above, a plurality of the illuminance sensors may be arranged in correspondence with the light-emitting devices respectively. The controller may compare sensed values of the illuminance sensors with each other. When a difference between the sensed values is greater than or equal to a predefined range, the controller may turn on or turn off corresponding light-emitting devices. For example, when some light-emitting devices face outwards and the other light-emitting devices face inwardly of the housing of the light source module as the substrate rotates, a detection value of the illuminance sensor corresponding to the light-emitting device facing outwards may have a great difference from a detection value of the illuminance sensor corresponding to the light-emitting device facing inwardly. Thus, the controller may compare the sensed values of the two illuminance sensors with each other to obtain a difference therebetween and may turn off the light-emitting device facing inwardly of the housing when the difference is greater than a predefined value. The light-emitting device facing inwardly of the housing may then be turned on in the same way when rotating and then facing outwardly.

After the controller 600 presets an illuminance range of the ambient light to at least one range, the controller may turn the light source 200 on or off, or adjust an amount of light from the light source 200, based on a detected illuminance range of the ambient light.

In one embodiment of the present disclosure, the illuminance sensor 320 may detect illuminance of external light. However, the light detected by the illuminance sensor 320 is not limited to external light. The illuminance sensor 320 may detect illuminance of light from the light source 200. In this case, when the illuminance of the light from the light source 200 is lower than a predefined range, the controller 600 may display a separate alarm for notifying replacement of the light source unit 210 on a separate display.

The light source module according to one embodiment of the present disclosure may be employed in various devices. For example, the light source module may be employed in kitchen furniture (sink) to sterilize tableware.

FIG. 12 shows an illumination device according to one embodiment of the present disclosure. The illumination device includes a light source member 20 to emit light of a predefined wavelength band, a casing 10 for receiving therein the light source member 20, and, a cover 30 to cover one side surface of the casing 10.

In FIG. 12, the light is emitted from the light source member 20 in a downward direction. In following descriptions, the emission direction of light is defined as a downward direction. An opposite direction thereto is defined an upward direction. However, the indication of this direction is set forth for convenience of description. The emission direction may be relatively different depending on the direction in which the illumination device is mounted on a specific device.

The casing 10 forms an appearance of the illumination device, and provides a space 19 where the light source member 20 is placed. Although the casing 10 forms the appearance of the illumination device, an additional casing or component may be provided outside the illumination device. Additional components may be further mounted when the light source member 20 is used in other devices.

The casing 10 generally corresponds to a shape of the light source member 20. One side of the casing may be opened so that light from the light source member 20 may travel therethrough.

An overall shape of the casing 10 may have a long bar shape as shown in FIG. 12, and the casing 10 may have an open bottom. In one embodiment of the present disclosure, the casing 10 may have a cuboidal shape as a whole and may include a bottom portion 11 and each side wall 15 connected to the bottom portion 11. However, the shape of the casing 10 may have various shapes, for example, a semi-cylindrical shape cut in a longitudinal direction as long as the casing 10 satisfies the concept of the present disclosure.

The bottom portion 11 may have a rectangular shape extending in an elongate manner in one direction. That is, the bottom portion 11 may be provided in an elongated plate shape having a horizontal plane. In one embodiment of the present disclosure, a portion of the light source member 20 may be disposed on the bottom portion 11.

The casing 10 may be made of various materials. The casing 10 may be made of a metal material such as aluminum, stainless, but is not limited thereto. The casing 10 may be made of other durable materials as long as the casing contains the light source member 20. The casing 10 may be made of polymer resin or a material that minimizes discoloration due to ultraviolet rays and cracking. When an entirety of the casing 10 is formed integrally, the casing 10 may be made of a single material, for example a metal. When at least a portion of the casing 10 is not integrally formed, the casing may be made of one material or two or more materials. For example, the bottom portion 11 and a portion of the side wall of the casing 10 may be made of a metal such as aluminum, while the remaining portion of the side wall may be made of polymer resin or the like.

Although not shown separately in the present embodiment, the bottom portion 11, and the side wall 15 may be equipped with a through-hole for extraction of a wire, and additional components such as a fastening structure for coupling the kitchen furniture and the like thereto. For example, one side of the bottom portion 11 may be provided with a through hole through which a wire for applying a power to the light source member 20 may pass. The wire may pass through the through-hole and connect to the light source member 20.

The light source member 20 is stored in the space 19 defined in the casing 10. The light source member 20 includes a first light source member 20a and a second light source member 20b that emit light beams of different wavelength bands respectively. The first light source member 20a emits visible light for lighting of a predefined region. The second light source member 100 is provided on the bottom portion 11 of the casing 10 to emit light for sterilization of a predefined region. In this case, the illumination device according to the above-described embodiment may be employed as the second light source member 20b.

The light emitted by the first light source member 20a may have various wavelength bands including a visible light region. Light from the first light source member 20a may include a visible wavelength band, and a wavelength band other than the visible wavelength band.

In one embodiment of the present disclosure, the first light source member 20a may be provided in a surface light source form and/or a point light source form. In one embodiment of the present disclosure, the first light source member 20a may be provided in a point-source form.

The first light source member 20a includes a first substrate 21a and at least one first light source 23 mounted on the first substrate 21a. In this embodiment, in an example, twelve first light source 23 are provided.

The first substrate 21a may be provided in a plate shape. The first substrate 21a may be extended in an elongate manner in a predefined direction. In this case, the predefined direction may be the same as the longitudinal direction of the casing 10. However, the shape of the first substrate 21a may not be limited thereto and may be provided in various forms in which the first light source 23 is mounted thereon.

At least one first light source 23, for example, a plurality of first light sources 23 may be disposed on one surface of the first substrate 21a. When the first light source 23 is provided in a plural manner, the first light sources 23 may be arranged in various shapes, for example, randomly arranged, arranged to have a specific shape, arranged along a straight line, or arranged along a zigzag shape. In this connection, the light sources may be arranged to radiate as much light as possible downwards.

When first light source member 20a includes a plurality of first light sources 23, the first light sources 23 may emit light of the same wavelength band or emit light beams of different wavelength bands respectively. For example, in one embodiment, all of the first light sources 23 may emit light in the visible wavelength band. In another one embodiment, some light sources may emit a portion of the visible wavelength band, while the remaining light sources may emit another portion of the visible wavelength range. When light beams from the first light sources 23 have different wavelength bands, the light sources may be arranged in various shapes and orders. For example, the first light sources 23 may emit visible light of various colors, for example, red, green, yellow, blue, etc. respectively. White light may be rendered by combining the first light sources 23 that emit light beams of various colors. Alternatively, white light may be rendered by combining a yellow phosphor on the first light source 23 that emits blue light. However, the wavelength band of the light emitted by the light source is not limited to the above range.

In this connection, when a light-emitting diode is used as the first light source 23 of the first light source member 20a, the light-emitting diode may be mounted on the first substrate 21a. The light-emitting diode may be installed in a form of an injection-type lead frame package such that the diode may be surface-mounted on the first substrate 21a, in a through hole mounting form, a bare chip form, or a flip chip form. Further, an additional substrate 21 may be attached thereto to improve heat dissipation or electrical properties of the light-emitting diode.

A connector (not shown) to connect the wire with the first light source 23 may be further provided on the first substrate 21a of the first light source member 20a. For example, a power wire may be connected to the first light source member 20a via the connector.

In one embodiment of the present disclosure, the first light source member 20a may provide light in a downward direction. When, as shown, the first light sources 23 are provided on one surface of the first substrate 21a, light may be mainly emitted in a direction perpendicular to a plane on which the first light sources 23 are provided. However, the direction of light emitted by the first light source member 20a may be variously modified.

As the second light source member 20b, an illumination device according to the embodiments described above may be employed. The second light source member 20b may emit germicidal light in the downward direction. That is, the second light source member 20b may emit light having a sterilization function to minimize growth of bacteria. In this connection, a portion of the second light source member 20b may be rotated to irradiate different regions with ultraviolet light to increase sterilization efficiency. In other words, in one embodiment, the second light source member 20b may be rotated at a predefined angle such that the light emitted from the second light source member 20b is provided in a direction different from the emission direction of the light from the first light source member 20a, for example, in the downward direction. The rotation angle of the second light source member 20b may vary depending on a position of a target to be sterilized.

In the above embodiment, although the second light source member 20b is fixedly disposed at a predefined position, the position of the second light source member 20b may be variously changed. In particular, the position of the second light source member 20b may be variously changed by the user.

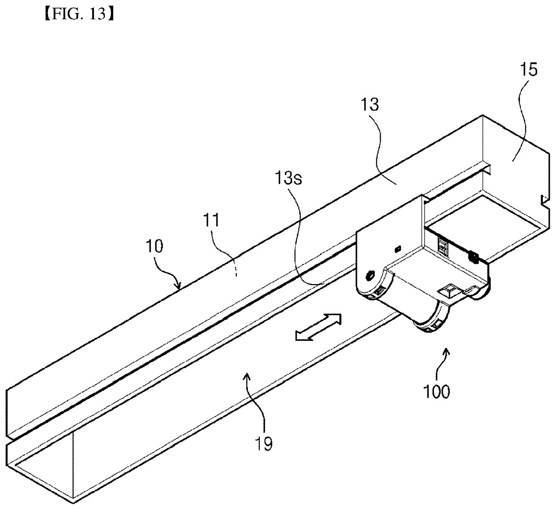

FIG. 13 is a perspective view showing an illumination device according to one embodiment of the present disclosure. In FIG. 13, the casing 10 and the second light source member 20b are mainly shown for clarity, and some components have been omitted.

Referring to FIG. 13, the illumination device according to the present embodiment may include detachable fastening means, such that the second light source member 20b may be fastened while changing a position thereof. The fastening means may be provided on at least one of the casing 10 and second light source member 20b. As long as the second light source member 20b may be combined with the predefined position of the casing 10, a type or shape of the fastening means is not limited. For example, in the present embodiment, the second light source member 20b and casing 10 may be equipped with a fastener 13s to fasten the second light source member 20b and casing 10 to each other. For example, the second light source member 20b may include a sliding protrusion that protrudes toward the casing 10 so that the second light source member 20b may be engaged with the casing 10. The casing 10 may be provided with a slide groove into which the sliding protrusion may be inserted. Accordingly, the second light source member 20b may be bound to the casing 10 and may move in the longitudinal direction of the casing 10 along the slide groove.

Although not shown separately, the second light source member 20b may be provided in a manual manner such that the user adjusts the position of the second light source member 20b directly or in an automatic electric control manner. According to the present embodiment, the emission position of the light from the second light source member 20b may be easily changed. In this case, ultraviolet rays may be irradiated to a wider region, which further widens the sterilization region. In this connection, although the embodiment shows that the light emission position of the second light source member 20b is changed along the length of the casing 10, the present disclosure is not limited thereto. Depending on a placement direction of the second light source member 20b, the light emission position may be changed in another direction, for example, along a width direction of the casing 10.

In the above-described embodiment, a slide is described as the detachable fastening means, but the fastening means is not limited thereto. The fastening means may be provided in various forms as long as the fastening means may be fastened to components in the illumination device at different positions.

Although described above with reference to the preferred embodiments of the present disclosure, it will be appreciated that those skilled in the art or those having a common knowledge in the art may make various modifications and changes to the present disclosure without departing from a spirit and scope of the present disclosure as described in the claims to be described later.

Therefore, a technical scope of the present disclosure should not be limited to the contents described in the detailed description of the specification but should be defined by the claims.

### [INDUSTRIAL APPLICABILITY]

The present disclosure may provide a light source module for various devices.

## Claims

1. A light source module comprising:
a light source unit including a substrate and a light-emitting device provided on the substrate;
a base to fix the substrate and rotatable around a pivot axis; and
a housing having a pivot ring surrounding the base, wherein the housing are coupled to the light source unit and the base.

2. The light source module of claim 1, wherein the light-emitting device has a orientation angle with respect to one direction, wherein the one direction changes within a predefined angle relative to the pivot axis.

3. The light source module of claim 1, wherein the substrate extends in one direction, wherein the base is provided at least one of both ends of the substrate and is coupled to the pivot ring.

4. The light source module of claim 3, wherein the pivot ring includes a stopper to limit an angle of rotation of the base.

5. The light source module of claim 4, wherein the stopper protrudes from the pivot ring toward the base, wherein the base has a rotatable movement-limiter, wherein a rotation range of the movement-limiter is limited by the stopper.

6. The light source module of claim 5, wherein the movement-limiter is partial-circle or arc shaped when viewed in a direction perpendicular to the pivot axis.

7. The light source module of claim 3, wherein the pivot ring has an opening, wherein the opening is formed by removing a portion of the pivot ring,
wherein the base includes a lever protruding in a direction perpendicular to the pivot axis and disposed within the opening,
wherein the lever is movable within the opening.

8. The light source module of claim 3, wherein the base includes a protrusion for rotation projecting outwards along the pivot axis.

9. The light source module of claim 1, wherein the light source module further comprises a protective tube containing the light source unit in the protective tube, wherein the protective tube protects the light source unit.

10. The light source module of claim 1, wherein the light source module further comprises a fastener mounted on the housing to fasten the light source module to an external component.

11. The light source module of claim 10, wherein the fastener includes a hook engaging in a sliding manner.

12. The light source module of claim 10, wherein the fastener includes a clip.

13. The light source module of claim 1, wherein the light source module further comprises a printed circuit substrate positioned adjacent to the light source unit, wherein a controller to drive the light source unit is mounted on the printed circuit substrate.

14. The light source module of claim 1, wherein the light source module further comprises a battery pack provided on one side of the housing to supply a power to the light source unit.

15. The light source module of claim 14, wherein the battery pack includes a charger rechargeable by an external power source.

16. The light source module of claim 15, wherein the battery pack includes a USB port to supply a power to the charger.

17. The light source module of claim 1, wherein the light source module further comprises a motion sensor mounted on the housing.

18. The light source module of claim 1, wherein the light source module further comprises an illuminance sensor mounted on the housing.

19. The light source module of claim 18, wherein the substrate has a plate shape having at least two planes, wherein the light-emitting device is mounted on at least one of the planes.

20. The light source module of claim 18, wherein the substrate is bent in different directions to define the at least two planes, wherein a plurality of light-emitting devices are mounted on the at least two planes respectively.

21. The light source module of claim 18, wherein the substrate is flexible.

22. The light source module of claim 1, wherein the light source module further comprises a controller configured to control a rotation of the light source unit and turn-on or turn-off of the light source unit.

23. The light source module of claim 22, wherein the light source module further comprises at least one of a motion sensor mounted on the housing or an illuminance sensor mounted on the housing,
wherein the controller is configured to control the rotation of the light source unit and the turn on/off of the light source unit based on a result detected by the at least one of the motion sensor or the illuminance sensor.

24. The light source module of claim 23, wherein the light source unit includes a plurality of light-emitting devices,
wherein each of a plurality of illuminance sensors corresponds to each of the light-emitting devices to detect external illuminance,
wherein the controller is configured to:
compare sensed values of the illuminance sensors with each other; and
when a difference between the sensed values of corresponding light-emitting devices is greater than or equal to a predefined range, turn on or off the corresponding light-emitting devices.

25. An illumination device comprising:
at least one first light source member to emit visible light;
at least one second light source member spaced apart from the first light source member and emitting light of a wavelength for sterilization; and
a casing housing the first light source and second light source members,
wherein the second light source member includes:
a light source unit including a substrate and a light-emitting device provided on the substrate;
a base to fix the substrate and rotatable around a pivot axis;
a housing having a pivot ring surrounding the base, wherein the housing are coupled to the light source unit and the base.

## Amended claims

### Amended claims under Art. 19.1 PCT

1. A light source module comprising:
a light source unit including a substrate extending in an elongated manner in one direction, and a light-emitting device provided on the substrate and emitting sterilizing light;
a protective tube housing the light source unit to protect the light source unit;
a printed circuit substrate positioned adjacent to the light source unit, wherein a controller to drive the light source unit is mounted on the printed circuit substrate;
a base for fixing the substrate and rotatable around a pivot axis; and
a housing having a pivot ring surrounding the base, wherein the housing are coupled to the light source unit and the base,
wherein the base is provided at least one of both ends of the substrate and is coupled to the pivot ring, wherein the pivot ring includes a stopper to limit an angle of rotation of the base.

2. The light source module of claim 1, wherein the light-emitting device has a direction angle with respect to one direction, wherein the one direction changes within a predefined angle relative to the pivot axis.

3. (deleted)

4. (deleted)

5. The light source module of claim 1, wherein the stopper protrudes from the pivot ring toward the base, wherein the base has a rotatable movement-limiter, wherein a rotation range of the movement-limiter is limited by the stopper.

6. The light source module of claim 5, wherein the movement-limiter is partial-circle or arc shaped when viewed in a direction perpendicular to the pivot axis.

7. The light source module of claim 1, wherein the pivot ring has an opening, wherein the opening is formed by removing a portion of the pivot ring,
wherein the base includes a lever protruding in a direction perpendicular to the pivot axis and disposed within the opening,
wherein the lever is movable within the opening.

8. The light source module of claim 1, wherein the base includes a rotating protrusion projecting outwards along the pivot axis.

9. (deleted)

10. The light source module of claim 1, wherein the light source module further comprises a fastener mounted on the housing to fasten the light source module to an external component.

11. The light source module of claim 10, wherein the fastener includes a hook engaging in a sliding manner.

12. The light source module of claim 10, wherein the fastener includes a clip.

13. (deleted)

14. The light source module of claim 1, wherein the light source module further comprises a battery pack provided on one side of the housing to supply a power to the light source unit.

15. The light source module of claim 14, wherein the battery pack includes a charger rechargeable by an external power source.

16. (deleted)

17. The light source module of claim 1, wherein the light source module further comprises a motion sensor mounted on the housing.

18. The light source module of claim 1, wherein the light source module further comprises an illuminance sensor mounted on the housing.

19. The light source module of claim 18, wherein the substrate has a plate having at least two planes, wherein the light-emitting device is mounted on at least one of the planes.

20. The light source module of claim 18, wherein the plate is bent in different directions to define the at least two planes, wherein a plurality of light-emitting devices are mounted on the at least two planes respectively.

21. The light source module of claim 18, wherein the substrate is flexible.

22. The light source module of claim 1, wherein the light source module further comprises a controller configured to control a rotation of the light source unit and turn-on or turn-off of the light source unit.

23. The light source module of claim 22, wherein the light source module further comprises at least one of a motion sensor mounted on the housing or an illuminance sensor mounted on the housing,
wherein the controller is configured to control the rotation of the light source unit and the turn on/off of the light source unit based on a result detected by the at least one of the motion sensor or the illuminance sensor.

24. The light source module of claim 23, wherein the light source unit includes a plurality of light-emitting devices,
wherein each of a plurality of illuminance sensors corresponds to each of the light-emitting devices to detect external illuminance,
wherein the controller is configured to:
compare sensed values the illuminance sensors with each other; and
when a difference between sensed values of corresponding light-emitting devices greater than or equal to a predefined range, turn on or off the corresponding light-emitting devices.

25. An illumination device comprising:
at least one first light source member to emit visible light;
at least one second light source member spaced apart from the first light source member and emitting light of a wavelength for sterilization; and
a casing housing the first light source and second light source members,
wherein the second light source member includes:
a light source unit including a substrate and a light-emitting device provided on the substrate;
a base for fixing the substrate and rotatable around a pivot axis;
a housing having a pivot ring surrounding the base, wherein the housing are coupled to the light source unit and the base.
